# FASCICULE DE BREVET EUROPEEN

(11) **EP 2 406 269 B1**
(45) Date de publication et mention de la délivrance du brevet: **26.02.2014**
(21) Numéro de dépôt: 10706667.2
(22) Date de dépôt: 05.03.2010
(51) Int. Cl.: C07F 9/6571, C07B 43/08, C07B 41/06, C07C 45/50

(54) **COMPOSES ORGANOPHOSPHORES, SYSTEMES CATALYTIQUES COMPRENANT CES COMPOSES ET PROCEDE D'HYDROCYANATION OU D'HYDROFORMYLATION UTILISANT CES SYSTEMES CATALYTIQUES**
PHOSPHORORGANISCHE VERBINDUNGEN, KATALYTISCHE SYSTEME, DIE DIESE VERBINDUNGEN UMFASSEN, UND HYDROCYANIERUNGS- ODER HYDROFORMYLIERUNGSVERFAHREN, BEI DEM DIESE KATALYTISCHEN SYSTEME ANGEWENDET WERDEN
ORGANOPHOSPHORUS COMPOUNDS, CATALYTIC SYSTEMS INCLUDING SAID COMPOUNDS AND HYDROCYANATION OR HYDROFORMYLATION METHOD USING SAID CATALYTIC SYSTEMS

(30) Priorité: 13.03.2009 FR 0951577
(43) Date de publication de la demande: 18.01.2012
(73) Titulaire: Rhodia Opérations, 93306 Aubervilliers (FR)
(72) Inventeur: MASTROIANNI, Sergio, F-69006 Lyon (FR); MIKHEL, Igor, 127 411 Moscow (RU); PRINGLE, Paul, Bristol Avon BS8 3DG (GB)
(86) Numéro de dépôt international: PCT/EP2010/052851
(87) Numéro de publication internationale: WO 2010/102962

(56) Documents cités:
- WO-A1-98/42717
- PUGH R I: "Phospha-adamantanes a new class of bulky alkyl phosphine ligands" THESIS,, 1 avril 2000 (2000-04-01), pages 47-97, XP008115606 School of Chemistry, University of Bristol
- VAN LEEUWEN ET AL: "Ligand Bite Angle Effects in Metal-catalyzed C-C Bond Formation"[Online] vol. 100, no. 8, 2000, pages 2741-2769, XP002585510 CHEM. REV. Extrait de l'Internet: URL:http://pubs.acs.org/doi/pdf/10.1021/cr 9902704> [extrait le 2010-06-30]
- BABER ET AL: "Phenylphosphatrioxa-adamantanes: bulky, robust, electron-poor ligands that give very efficient rhodium(I) hydroformylation catalysts" DALTON TRANS., 2005, pages 1079-1085, XP002561939
- EPSTEIN ET AL: "A novel phosphorus heterocyclic system from the reactions of phosphine and primary phosphines with 2,4-pentanedione" J. AM. CHEM. SOC., vol. 83, 1961, pages 3279-3292, XP002561940
- DOWNING ET AL: "GENERAL ROUTES TO ALKYL PHOSPHATRIOXAADAMANTANE LIGANDS" ORGANOMETALLICS, vol. 27, 11 juin 2008 (2008-06-11), pages 3216-3224, XP002561941
- J P Hopewell: "Phospha-adamantane Cage Ligands: Their Resolution, Organometallic Chemistry and Applications in Catalysis", 2008 Student Conference Applied Catalysis: Towards Sustainable Chemical Industry 12 November 2008, 12 November 2008 (2008-11-12), XP55042819, Retrieved from the Internet: URL:http://www.bath.ac.uk/csct/news/IChemE _Catalysis_2008.html [retrieved on 2012-10-31]
- JONATHAN PAUL HOPEWELL: "New directions in phospha-adamantane chemistry", A THESIS SUBMITTED TO THE UNIVERSITY OF BRISTOL IN ACCORDANCE WITH THE REQUIREMENTS OF THE DEGREE OF DOCTOR OF PHILOSOPHY IN THE SCHOOL OF CHEMSITRY, FACULTY OF SCIENCE, UNIVERSITY OF BRISTOL, GB, 1 August 2009 (2009-08-01), pages I-xv,1, XP008127653,

## Description

La présente invention concerne des composés organophosphorés appartenant à la famille des phosphinites, leur utilisation dans des systèmes catalytiques et les procédés de synthèse de composés organiques utilisant ces systèmes catalytiques, notamment les procédés d'hydrocyanation de composés organiques à insaturation éthylénique en composés comprenant au moins une fonction nitrile et les procédés d'hydroformylation de composés insaturés pour la fabrication d'aldéhydes.

La réaction d'hydrocyanation est, par exemple, décrite dans le brevet français n° 1 599 761 qui concerne un procédé de préparation de nitriles par addition d'acide cyanhydrique sur des composés organiques ayant au moins une double liaison éthylénique, en présence d'un catalyseur comprenant du nickel et un ligand organophosphorés, une triarylphosphite. Cette réaction peut être conduite en présence ou non d'un solvant.

Quand un solvant est utilisé, il s'agit de préférence d'un hydrocarbure, tel que le benzène ou les xylènes ou d'un nitrile tel que l'acétonitrile.

Le catalyseur mis en oeuvre est un complexe organique de nickel, contenant des ligands tels que les phosphines, les arsines, les stibines, les phosphites, les arsénites ou les antimonites.

La présence d'un promoteur pour activer le catalyseur, tel qu'un composé du bore ou un sel métallique, généralement un acide de Lewis, est également préconisée dans ledit brevet.

De nombreux autres systèmes catalytiques ont été proposés comprenant généralement des composés organophosphorés appartenant à la famille des phosphites, phosphonites, phosphinites et phosphines. Ces composés organophosphorés peuvent comprendre un atome de phosphore par molécule et sont qualifiés de ligands monodentates, ou plusieurs atomes de phosphore par molécules, ils sont alors appelés ligands pluridentate. Plus particulièrement de nombreux ligands contenant deux atomes de phosphore par molécule (ligand bidentate) ont été décrits dans de nombreux brevets.

Le document WO9842717 décrit des ligands diphosphines utilisés dans des systèmes catalytiques pour un procédé de carbonylation d'alcènes.
La thèse « Phospha-adamantane a new class of bulky alkyl phosphine ligands » (1 avril 2000, School of chemistry, University of Bristol) de Pugh R.I décrit des ligants phosphines.

Toutefois, la recherche de nouveaux systèmes catalytiques plus performants tant en activité catalytique qu'en stabilité est toujours entreprise pour améliorer l'économie générale du procédé.

La réaction d'hydroformylation, qui consiste à faire réagir un composé oléfinique avec un mélange d'oxyde de carbone et d'hydrogène sous pression pour former un aldéhyde, est également décrite dans de nombreux documents. On peut, par exemple, avoir une vue générale de l'hydroformylation des oléfines dans B. Cornils and W.A. Herrmann (Eds.), Applied Homogeneous Catalysis with Organometallic Compounds, Vol.1 et 2, Weinheim, 1996 et dans l'article de E. KUNTZ publié dans l'encyclopédie « Les Techniques de l'Ingénieur » J 5 750-1 édition de 2002. Les systèmes catalytiques utilisés dans cette réaction comprennent généralement des hydrures de cobalt ou rhodium carbonylés pouvant être complexés par un ligand organophosphoré, notamment des organophosphines telles que la triphénylphosphine ou des organophosphites. Une description générale des systèmes catalytiques utilisés dans l'hydroformylation des alcènes est donnée dans le brevet US7495133. Comme pour l'hydrocyanation, de nouveaux systèmes catalytiques sont recherchés pour améliorer les propriétés du procédé tant au niveau des performances du catalyseur que de la simplification du procédé.

Un des buts de la présente invention est de proposer une nouvelle famille de ligands qui permet d'obtenir avec les métaux de transition des systèmes catalytiques présentant une bonne activité catalytique notamment dans les réactions d'hydrocyanation et d'hydroformylation.

A cet effet, la présente invention propose des composés organophosphorés, caractérisés en ce que ces composés organophosphorés correspondent à l'une des formules générales (I) et (II): 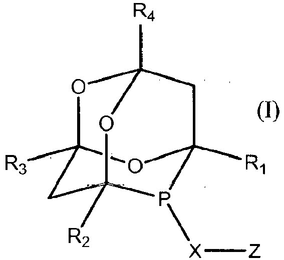 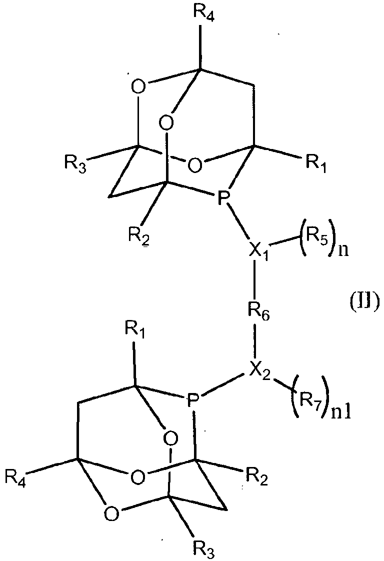 dans lesquelles :
- R₁, R₂, R₃, R₄, R₅, R₇ et Z identiques ou différents représentent un atome d'hydrogène, un radical alkyl linéaire ou ramifié ayant de 1 à 12 carbones pouvant contenir des hétéroatomes, un radicale comprenant un radical aromatique ou cycloaliphatique substitué ou non pouvant comprendre des hétéroatomes, un radical carbonyle, alcoxycarbonyle ou alcoxy, un atome d'halogène, un groupement nitrile ou un groupement halogénoalkyle ayant 1 à 12 atomes de carbone
- X, X1 et X2 identiques ou différents représentent un atome d'oxygène, d'azote, de soufre, ou de silicium
- R₆ représente une liaison covalente, un radical aliphatique linéaire ou ramifié, un radical comprenant un cycle aromatique ou cycloaliphatique substitué ou non ou plusieurs cycles aromatiques condensés ou reliés entre eux par une liaison
- n et n1 identiques ou différents sont des nombres entiers respectivement égaux à la valence des éléments X₁, X₂ diminuée de 2.

Selon un mode de réalisation particulier de l'invention, ces composés organophosphorés appartiennent à la famille des organophosphinites et correspondent à l'une des formules générales (III) et (IV): 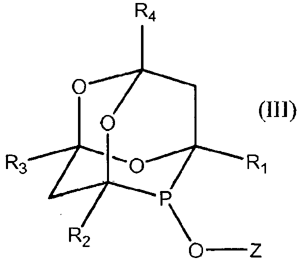 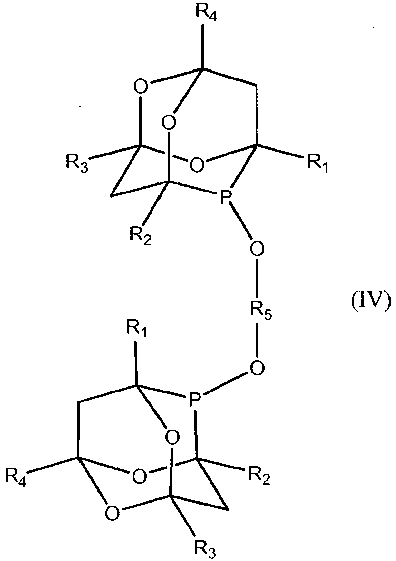 dans lesquelles :
- R₁, R₂, R₃, R₄ identiques ou différents représentent un atome d'hydrogène, un radical alkyl linéaire ou ramifié ayant de 1 à douze atomes de carbone pouvant contenir des hétéroatomes,
- Z représente un radical aromatique ou cycloaliphatique substitué ou non pouvant comprendre des hétéroatomes, un radical carbonyle, alcoxycarbonyle ou alcoxy, un atome d'halogène, un groupement nitrile ou un groupement halogénoalkyle ayant un à douze atomes de carbone,
- R₅ représente une liaison covalente, un radical aliphatique linéaire ou ramifié, un radical comprenant un cycle aromatique ou cycloaliphatique substitué ou non ou plusieurs cycles aromatiques condensés ou reliés entre eux par une liaison.

Comme composés de formule générale (I), (II), (III) ou (IV) préférés de l'invention, on peut citer les composés de formules suivantes :

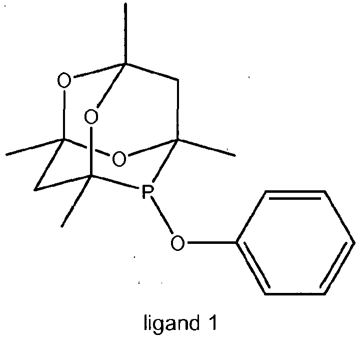

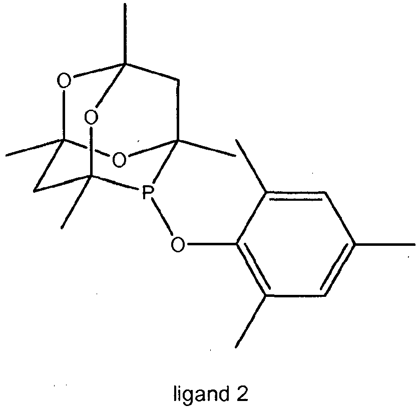

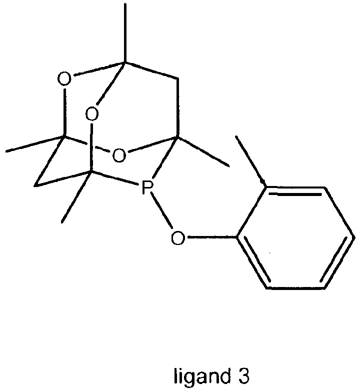

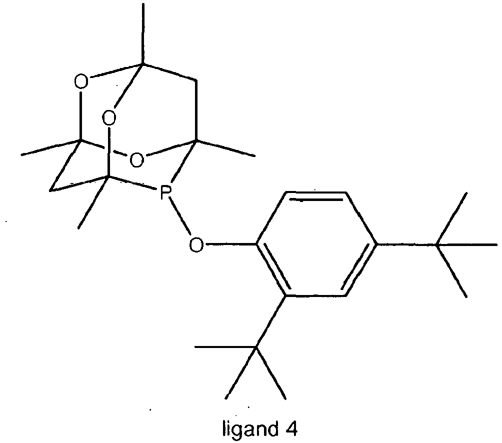

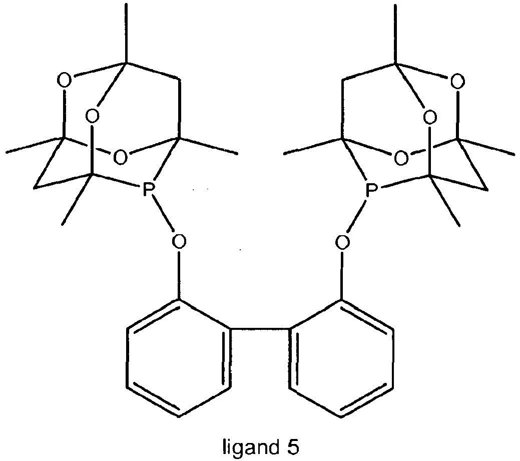

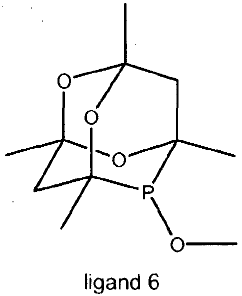

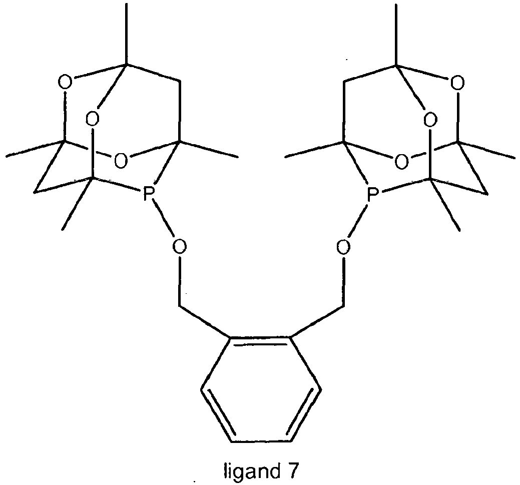

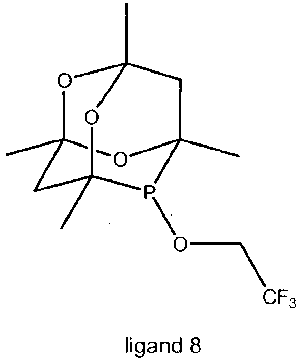

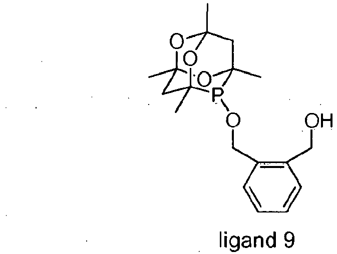

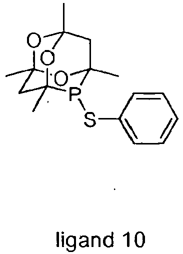

Ces composés peuvent être préparés à partir de composés appelés ci-après CgPH de formule suivante :

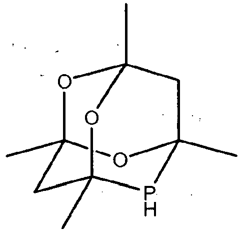

Ces composés CgPH sont décrits dans l'article scientifique publié dans la revue ORGANOMETALLICS vol 27, N° 13 p 3215-3224 de 2008: « General Routes to Alkyl Phosphotrioadamantane Ligands » de Joanne H. Downing et al, ainsi que leur procédé de synthèse.

Les composés CgPH sont transformés en composés CgPX, dans laquelle X représente un atome d'halogène, de préférence le brome, par réaction avec l'halogène moléculaire dans un solvant organique tel que le dichlorométhane.

Les composés CgPX sont ensuite mis en réaction par exemple avec un composé obtenu par réaction d'un composé hydroxylé correspondant au reste Z ou R5 des formules générales III et IV avec un composé organoalcalin, de préférence organolithien, dans un solvant tel que le tétrahydrofurane.

Des détails et informations complémentaires sur les procédés de fabrication des composés de formules I et II seront donnés dans les exemples ci-après.

Selon un autre objet de l'invention, les composés organophosphorés de formules (I), (II), (III) ou (IV) sont utilisés pour la fabrication de système catalytique par association avec un élément métallique pour former un complexe. Globalement, la composition de ces systèmes catalytiques peut être représentée par la formule générale (V) ou (VI) (ces formules ne correspondent pas à la structure des composés et complexes présents dans le système catalytique):

M [L_{f}]ₜ (V)

ou

HM (L_{f}]ₜ₊ₙ(CO)₄₋ₙ (VI)

dans laquelle:
M est un métal de transition
L_{f} représente au moins un ligand organophosphoré de formule (I), (II), (III) ou (IV)
t représente un nombre compris entre 1 et 10 (bornes incluses)
n représente un nombre compris entre 1 et 4 (bornes incluses)
   a. Les métaux M qui peuvent être complexés sont de manière générale tous les métaux de transition des groupes 1 b, 2b, 3b, 4b, 5b, 6b, 7b et 8 de la Classification périodique des éléments, telle que publiée dans "Handbook of Chemistry and Physics, 51st Edition (1970-1971)" de The Chemical Rubber Company.

Parmi ces métaux, on peut citer plus particulièrement les métaux pouvant être utilisés comme catalyseurs dans les réactions d'hydrocyanation et d'hydroformylation. Ainsi on peut mentionner à titre d'exemples non limitatifs, le nickel, le cobalt, le fer, le ruthénium, le rhodium, le palladium, l'osmium, l'iridium, le platine, le cuivre, l'argent, l'or, le zinc, le cadmium, le mercure. Le nickel est l'élément préféré pour l'hydrocyanation des oléfines et des nitriles insaturés et le cobalt ou le rhodium pour l'hydroformylation notamment des alcènes.

La préparation des systèmes catalytiques comprenant des composés de formule générale (I), (II), (III) ou (IV) peut être effectuée en mettant en contact une solution d'un composé du métal choisi, par exemple le nickel ou le rhodium, avec une solution du composé organophosphoré de l'invention.

Le composé du métal peut être dissous dans un solvant. Le métal peut se trouver dans le composé mis en oeuvre, soit au degré d'oxydation qu'il aura dans le complexe organométallique, soit à un degré d'oxydation supérieur.

A titre d'exemple, on peut indiquer que dans les complexes organométalliques de l'invention, le rhodium est au degré d'oxydation (I), le ruthénium au degré d'oxydation (II), le platine au degré d'oxydation (0), le palladium au degré d'oxydation (0), l'osmium au degré d'oxydation (II), l'iridium au degré d'oxydation (I), le nickel au degré d'oxydation (0).

Si lors de la préparation du complexe organométallique, le métal est mis en oeuvre à un degré d'oxydation plus élevé, il pourra être réduit in situ.

Parmi les composés de métaux M utilisables pour la préparation des complexes organométalliques, notamment quand le métal est le nickel, on peut citer, à titre d'exemples non limitatifs, les composés suivants du nickel :
- les composés dans lesquels le nickel est au degré d'oxydation zéro comme le tétracyanonickelate de potassium K₄ [Ni(CN)₄], le bis (acrylonitrile) nickel zéro, le bis (cyclooctadiène-1,5) nickel (appelé également Ni(cod)₂ ) et les dérivés contenant des ligands comme le tétrakis (triphényl phosphine) nickel zéro.
- les composés du nickel comme les carboxylates (notamment l'acétate), carbonate, bicarbonate, borate, bromure, chlorure, citrate, thiocyanate, cyanure, formiate, hydroxyde; hydrophosphite, phosphite, phosphate et dérivés, iodure, nitrate, sulfate, sulfite, aryl- et alkyl-sulfonates.

Quand le composé du nickel utilisé correspond à un état d'oxydation du nickel supérieur à 0, on ajoute au milieu réactionnel un réducteur du nickel réagissant préférentiellement avec celui-ci dans les conditions de la réaction. Ce réducteur peut être organique ou minéral. On peut citer comme exemples non limitatifs les borohydrures comme le BH₄Na, le BH₄K, la poudre de Zn, le magnésium ou l'hydrogène.

Quand le composé du nickel utilisé correspond à l'état d'oxydation 0 du nickel, on peut également ajouter un réducteur du type de ceux précités, mais cet ajout n'est pas impératif.

Quand on utilise un composé du fer, les mêmes réducteurs conviennent. Dans le cas du palladium, les réducteurs peuvent être, en outre, des éléments du milieu réactionnel (phosphine, solvant, oléfine).

Quand le métal utilisé est le rhodium, pour la préparation des systèmes catalytiques comprenant des composés de formule générale (I), (II), (III) ou (IV), on peut préparer les complexes en utilisant comme composés du rhodium les composés de formule (acac)Rh (CO)₂ ou (acac)Rh(COD), dans lesquelles :
- acac signifie: acétylacétonate
- COD signifie: cyclooctadiène

La présente invention a également pour objet un procédé d'hydrocyanation d'oléfines, plus particulièrement de dioléfines pour la fabrication de composés nitriles, plus particulièrement de composés dinitriles.

Les composés organiques comportant au moins une double liaison éthylénique plus particulièrement mis en oeuvre dans le présent procédé sont des dioléfines comme le butadiène, l'isoprène, l'hexadiène-1,5, le cyclooctadiène-1,5, les nitriles aliphatiques à insaturation éthylénique, particulièrement les pentène-nitriles linéaires comme le pentène-3-nitrile, le pentène-4-nitrile et également les monooléfines comme le styrène, le méthyl-styrène, le vinyl-naphtalène, le cyclohexène, le méthyl-cyclohexène ainsi que les mélanges de plusieurs de ces composés.

Les pentène-nitriles peuvent contenir, en plus du pentène-3-nitrile et du pentène-4-nitrile, des quantités, généralement minoritaires, d'autres composés, comme le méthyl-2-butène-3-nitrile, le méthyl-2-butène-2-nitrile, le pentène-2-nitrile, le valéronitrile, l'adiponitrile, le méthyl-2-glutaronitrile, l'éthyl-2-succinonitrile ou le butadiène, provenant par exemple de la réaction antérieure d'hydrocyanation du butadiène en nitriles insaturés.

En effet, lors de l'hydrocyanation du butadiène, il se forme avec les pentène-nitriles linéaires des quantités non négligeables de méthyl-2-butène-3-nitrile et de méthyl-2-butène-2-nitrile.

Le système catalytique utilisé pour l'hydrocyanation selon le procédé de l'invention peut être préparé avant son introduction dans la zone de réaction, par exemple par addition au composé de formule (I), (II), (III) ou (IV) seul ou dissous dans un solvant, la quantité appropriée de composé du métal de transition choisi et éventuellement du réducteur. Il est également possible de préparer le système catalytique "in situ" par simple addition du composé de formule (I), (II), (III) ou (IV) et du composé du métal de transition dans le milieu réactionnel d'hydrocyanation avant ou après l'addition du composé à hydrocyaner.

La quantité de composé du nickel ou d'un autre métal de transition utilisée est choisie pour obtenir une concentration en mole de métal de transition par mole de composés organiques à hydrocyaner ou isomériser comprise entre 10⁻⁴ et 1, et de préférence entre 0,005 et 0,5 mole de nickel ou de l'autre métal dé transition mis en oeuvre.

La quantité de composé de formule (I), (II), (III) ou (IV) utilisée pour former le catalyseur est choisie de telle sorte que le nombre de moles de ce composé rapporté à 1 mole de métal de transition soit de 0,5 à 100 et de préférence de 2 à 50.

Bien que la réaction soit conduite généralement sans solvant, il peut être avantageux de rajouter un solvant organique inerte. Le solvant peut être un solvant du catalyseur qui est miscible à la phase comprenant le composé à hydrocyaner à la température d'hydrocyanation. A titre d'exemples de tels solvants, on peut citer les hydrocarbures aromatiques, aliphatiques ou cycloaliphatiques.

La réaction d'hydrocyanation est généralement réalisée à une température de 10°C à 200°C et de préférence de 30°C à 120°C. Elle peut être réalisée en milieu monophasique.

Le procédé d'hydrocyanation de l'invention peut être mis en oeuvre de manière continue ou discontinue.

Le cyanure d'hydrogène mis en oeuvre peut être préparé à partir des cyanures métalliques, notamment le cyanure de sodium, ou des cyanhydrines, comme la cyanhydrine de l'acétone ou par tout autre procédé de synthèse connu tel que le procédé Andrussov consistant à faire réagir le méthane avec l'ammoniac et de l'air.

Le cyanure d'hydrogène exempt d'eau est introduit dans le réacteur sous forme gazeuse ou sous forme liquide. Il peut également être préalablement dissous dans un solvant organique.

Dans le cadre d'une mise en oeuvre discontinue, on peut en pratique charger dans un réacteur, préalablement purgé à l'aide d'un gaz inerte (tel qu'azote, argon), soit une solution contenant la totalité ou une partie des divers constituants tels que le composé de formule I, II, III ou IV, le composé de métal de transition (le nickel), les éventuels réducteur et solvant, soit séparément lesdits constituants. Généralement le réacteur est alors porté à la température choisie, puis le composé à hydrocyaner est introduit. Le cyanure d'hydrogène est alors lui-même introduit, de préférence de manière continue et régulière.

Quand la réaction (dont on peut suivre l'évolution par dosage de prélèvements) est terminée, le mélange réactionnel est soutiré après refroidissement et les produits de la réaction sont isolés et séparés, par exemple, par distillation.

Avantageusement, la synthèse de dinitriles tels que l'adiponitrile à partir de dioléfines (butadiène) est obtenue en deux étapes successives. La première étape consiste à hydrocyaner une double liaison de la dioléfine pour obtenir un mononitrile insaturé. La seconde étape consiste à hydrocyaner l'insaturation du mononitrile pour obtenir le ou les dinitriles correspondants. Ces deux étapes sont généralement mises en oeuvre avec un système catalytique comprenant un complexe organométallique de même nature. Toutefois, les rapports composé organophosphoré/ élément métallique et concentration du catalyseur peuvent être différents. En outre, il est préférable d'associer au système catalytique un cocatalyseur ou promoteur dans la seconde étape. Ce cocatalyseur ou promoteur est généralement un acide de Lewis.

L'acide de Lewis utilisé comme cocatalyseur permet notamment, dans le cas de l'hydrocyanation des nitriles aliphatiques à insaturation éthylénique, d'améliorer la linéarité des dinitriles obtenus, c'est-à-dire le pourcentage de dinitrile linéaire par rapport à la totalité des dinitriles formés, et/ou d'augmenter l'activité et la durée de vie du catalyseur.

Par acide de Lewis, on entend dans le présent texte, selon la définition usuelle, des composés accepteurs de doublets électroniques.

On peut mettre en oeuvre notamment les acides de Lewis cités dans l'ouvrage édité par G.A. OLAH "Friedel-Crafts and related Reactions", tome I, pages 191 à 197 (1963).

Les acides de Lewis qui peuvent être mis en oeuvre comme cocatalyseurs dans le présent procédé sont choisis parmi les composés des éléments des groupes Ib, IIb, IIIa, IIIb, IVa, IVb, Va, Vb, Vlb, Vllb et VIII de la Classification périodique des éléments. Ces composés sont le plus souvent des sels, notamment des halogénures, comme chlorures ou bromures, sulfates, sulfonates, halogénosulfonates, perhalogénoalkyl sulfonates, notamment fluoroalkylsulfonates ou perfluoroalkylsulfonates, carboxylates et phosphates.

A titre d'exemples non limitatifs de tels acides de Lewis, on peut citer le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le trifluorométhylsulfonate d'indium, les chlorures ou bromures des éléments des terres rares comme le lanthane, le cérium, le praséodyme, le néodyme, le samarium, l'europium, le gadolinium, le terbium, le dysprosium, l'hafnium, l'erbium, le thallium, l'ytterbium et le lutétium, le chlorure de cobalt, le chlorure ferreux, le chlorure d'yttrium.

On peut également utiliser comme acide de Lewis des composés organométalliques comme le triphénylborane, l'isopropylate de titane ou les composés décrits dans la demande de brevet français non publiée déposée le 25 janvier 2008 sous le n°08 00381.

On peut bien entendu mettre en oeuvre des mélanges de plusieurs acides de Lewis.

Parmi les acides de Lewis, on préfère tout particulièrement le chlorure de zinc, le bromure de zinc, le chlorure stanneux, le bromure stanneux le triphénylborane et les mélanges chlorure de zinc/chlorure stanneux.

Le cocatalyseur acide de Lewis mis en oeuvre représente généralement de 0,01 à 50 moles par mole de composé de métal de transition, plus particulièrement de composé du nickel, et de préférence de 1 à 10 mole par mole.

Les mononitriles insaturés mis en oeuvre dans cette deuxième étape sont avantageusement des pentène-nitriles linéaires comme le pentène-3-nitrile, le pentène-4-nitrile et leurs mélanges.

Ces pentène-nitriles peuvent contenir des quantités, généralement minoritaires, d'autres composés, comme le méthyl-2-butène-3-nitrile, le méthyl-2-butène-2-nitrile, le pentène-2-nitrile.

La solution catalytique utilisée pour l'hydrocyanation en présence d'acide de Lewis peut être préparée avant son introduction dans la zone de réaction, par exemple par addition au composé de formule (I), (II), (III) ou (IV), de la quantité appropriée de composé du métal de transition choisi, de l'acide de Lewis et éventuellement du réducteur. Il est également possible de préparer la solution catalytique "in situ" par simple addition de ces divers constituants dans le milieu réactionnel.

Il est également possible dans les conditions du procédé d'hydrocyanation de la présente invention, et notamment en opérant en présence du système catalytique décrit précédemment comportant un composé de formule (I), (II), (III) ou (IV) et au moins un composé d'un métal de transition, de réaliser, en absence de cyanure d'hydrogène, l'isomérisation du méthyl-2-butène-3-nitrile en pentènenitriles, et plus généralement des nitriles insaturés ramifiés en nitriles insaturés linéaires.

Le méthyl-2-butène-3-nitrile soumis à l'isomérisation selon l'invention peut être mis en oeuvre seul ou en mélange avec d'autres composés. Ainsi on peut engager du méthyl-2-butène-3-nitrile en mélange avec du méthyl-2-butène-2 nitrile, du pentène-4-nitrile, du pentène-3-nitrile, du pentène-2-nitrile, du butadiène.

II est particulièrement intéressant de traiter le mélange réactionnel provenant de l'hydrocyanation du butadiène par HCN en présence d'au moins un composé de formule (I), (II). (III) ou (IV) et d'au moins un composé d'un métal de transition, plus préférentiellement d'un composé du nickel au degré d'oxydation 0, tel que défini précédemment. Dans le cadre de cette variante préférée, le système catalytique étant déjà présent pour la réaction d'hydrocyanation du butadiène, il suffit d'arrêter toute introduction de cyanure d'hydrogène, pour laisser se produire la réaction d'isomérisation.

On peut, le cas échéant, dans cette variante faire un léger balayage du réacteur à l'aide d'un gaz inerte comme l'azote ou l'argon par exemple, afin de chasser l'acide cyanhydrique qui pourrait être encore présent.

La réaction d'isomérisation est généralement réalisée à une température comprise entre 10°C et 200°C et de préférence entre 60°C et 140°C.

Dans le cas préféré d'une isomérisation suivant immédiatement la réaction d'hydrocyanation du butadiène, il sera avantageux d'opérer à la température à laquelle l'hydrocyanation a été conduite ou légèrement supérieure.

Comme pour le procédé d'hydrocyanation de composés à insaturation éthylénique, le système catalytique utilisé pour l'isomérisation peut être préparé avant son introduction dans la zone de réaction, par exemple par mélange du composé de formule (I), (II), (III) ou (IV), de la quantité appropriée de composé du métal de transition choisi et éventuellement du réducteur. Il est également possible de préparer le système catalytique "in situ" par simple addition de ces divers constituants dans le milieu réactionnel. La quantité de composé du métal de transition et plus particulièrement du nickel utilisée, ainsi que la quantité de composé de formule (I), (II), (III) ou (IV) sont les mêmes que pour la réaction d'hydrocyanation.

Bien que la réaction d'isomérisation soit conduite généralement sans solvant, il peut être avantageux de rajouter un solvant organique inerte qui pourra être utilisé comme solvant d'extraction, ultérieurement. C'est notamment le cas lorsqu'un tel solvant a été mis en oeuvre dans la réaction d'hydrocyanation du butadiène ayant servi à préparer le milieu soumis à la réaction d'isomérisation. De tels solvants peuvent être choisis parmi ceux qui ont été cités précédemment pour l'hydrocyanation.

Toutefois, la préparation de composés dinitriles par hydrocyanation d'une oléfine comme le butadiène peut être réalisée en utilisant un système catalytique conforme à l'invention pour les étapes de formation des nitriles insaturés et l'étape d'isomérisation ci-dessus, la réaction d'hydrocyanation des nitriles insaturés en dinitriles pouvant être mise en oeuvre avec un système catalytique conforme à l'invention ou tout autre système catalytique déjà connu pour cette réaction.

De même, la réaction d'hydrocyanation de l'oléfine en nitriles insaturés et l'isomérisation de ceux-ci peuvent être réalisées avec un système catalytique différent de celui de l'invention, l'étape d'hydrocyanation des nitriles insaturés en dinitriles étant mise en oeuvre avec un système catalytique conforme à l'invention.

L'invention a également pour objet un procédé d'hydroformylation d'alcènes pour la synthèse d'aldéhydes.

Les alcènes mis en oeuvre dans le présent procédé sont, à titre d'exemple, les oléfines linéaires comme l'éthylène, le propène, le 1-butène, le 1-pentène, le 1-hexène, le 1-octène.

De façon générale la réaction d'hydroformylation en présence de catalyseur à base de complexes de rhodium et de composés organophosphorés est décrite, par exemple, dans l'article de J. FALBE, « New syntheses with carbon monoxide », Springer Verlag, Berlin, Heidelberg, new York, pages 95 et suivantes (1980). Les oléfines réagissent avec un mélange CO/H₂ ( gaz de synthèse) en présence d'un catalyseur. La réaction est réalisée dans des domaines de température entre 40 à 180°C, de préférence entre 60°C et 140°C, sous une pression comprise entre 1 et 300 bar, de préférence entre 10 et 70 bar. Le mélange CO/H₂ (gaz de synthèse) présente un rapport volumique hydrogène /monoxyde de carbone compris entre 1 et 1,25. Le catalyseur et le ligand sont dissous dans le milieu d'hydroformylation qui peut, éventuellement comprendre un solvant.

D'autres détails, avantages de l'invention seront illustrés par les exemples donnés ci-dessous uniquement à titre indicatif, sans caractère limitatif.

### EXEMPLES

### Abréviations utilisées

- Ph : radical phényle
- Cod: cyclooctadiène
- Ni(Cod)₂ : bis(1,5-cyclooctadiène)nickel
- Rh(acac)(CO)2 : rhodium acétylacétonate dicarbonyle
- 3PN:3-pentènenitrile
- AdN: Adiponitrile
- ESN: éthylsuccinonitrile
- MGN: méthylglutaronitrile
- DN : composés dinitriles (AdN, MGN ou ESN)
- TI BAO: tetraisobutyldialuminoxane
- TT(Y) : taux de transformation du produit à transformer Y correspondant au rapport du nombre de moles transformées de Y sur le nombre de moles initiales de Y
- RR(DN): rendement réel de dinitriles correspondant au rapport du nombre de moles formées de dinitriles sur le nombre de moles de 3PN engagé
- Linéarité (L): rapport du nombre de moles d'AdN formées sur le nombre de moles de dinitriles formées (somme des moles d'AdN, ESN et MGN)

Les composés suivants: 3PN, Ni(Cod)₂, ZnCl₂, TIBAO, BPh₃, anhydride diphénylborinique (Ph₂BOPh₂), Rh(acac)(CO)₂ sont des produits connus et disponibles commercialement.

### Exemples 1 à 10 : Préparation des ligands 1 à 10

Dans une première étape, une solution de Br₂ (3,5158g, 0,022 mol) dans CH₂Cl₂ (30 ml) est ajoutée pendant 30 minutes à une solution de CgPH (4,3243 g, 0,02 mol) dans 60 ml de dichlorométhane (CH₂Cl₂) à 0°C et agitée à cette température pendant 30 minutes, puis pendant une heure à température ambiante. Le solvant est évaporé et un solide légèrement jaune est obtenu (CgPBr). RMN ³¹P δ 53,5 (dans CH₂Cl₂) :

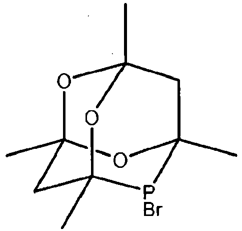

### Composé CgPBr

### Fabrication du ligand 1 :

Fabrication Une solution de butyle lithium (BuLi) dans l'hexane (1,6 M, 0;02 mol, 12,5 ml) et ajoutée lentement à une solution de phénol (11,8822 g, 0,02 mol) dans le tétrahydrofuranne (THF) (20 ml) à 0°C. Le mélange est agité pendant 1 heure à température ambiante puis ajouté pendant 30 min à une solution de CgPBr (5,9022 g, 0,02 mol) dans le THF (60 ml) à 0°C. La suspension obtenue est agitée toute une nuit avant évaporation du solvant. Une solution du ligand brut dans CH₂Cl₂ est filtrée sur silice et le solvant est évaporé. Le ligand est purifié par chromatographie sur colonne de silice en utilisant comme solvant un mélange CH₂Cl₂/Pentane (1/3 en volume) sous pression d'azote.
Quantité obtenue : 4,33 g (rendement : 70%)
RMN ³¹P δ 79,3 (dans CDCl₃)

### Fabrication du ligand 2 :

Une solution de BuLi dans l'hexane (1,6 M, 0,02 mol, 12,5 ml) et ajoutée lentement à une solution de 2,4,6-trimethylphénol (2,7238 g, 0,02 mol) dans le THF (20 ml) à 0°C. Le mélange est agité pendant 1 heure à température ambiante puis ajouté pendant 30 min à une solution de CgPBr (5,9022 g, 0,02 mol) dans le THF (60 ml) à 0°C. La suspension obtenue est agitée toute une nuit avant évaporation du solvant. Le ligand brut est dissous dans CH₂Cl₂ (60 ml) et 50 ml d'eau sont ajoutés. La phase aqueuse est extraite avec du CH₂Cl₂ (50 ml) et l'ensemble des phases organiques est séché sur Na₂SO₄, filtré et le solvant est évaporé. Le ligand est purifié par chromatographie sur colonne de silice en utilisant comme solvant un mélange CH₂Cl₂/Pentane (1/3 en volume) sous pression d'azote.
Quantité obtenue : 4,21 g (60%)
RMN ³¹P δ 82,0 (dans CDCl₃)

### Fabrication du ligand 3 :

Une solution de BuLi dans l'hexane (1,6 M, 0,02 mol, 12,5 ml) et ajoutée lentement à une solution de 2-methylphénol (2,1628 g, 0,02 mol) dans le THF (20 ml) à 0°C. Le mélange est agité pendant 1 heure à température ambiante puis ajouté pendant 30 min à une solution de CgPBr (5,9022 g, 0,02 mol) dans le THF (60 ml) à 0°C. La suspension obtenue est agitée toute une nuit avant évaporation du solvant. Le ligand brut est dissous dans CH₂Cl₂ (60 ml) et 50 ml d'eau sont ajoutés. La phase aqueuse est extraite avec du CH₂Cl₂ (50 ml) et l'ensemble des phases organiques est séché sur Na₂SO₄, filtré et le solvant est évaporé. Le ligand est purifié par chromatographie sur colonne de silice en utilisant comme solvant un mélange CH₂Cl₂/Pentane (1/3 en volume) sous pression d'azote.
Quantité obtenue : 5,01 g (78%)
RMN ³¹P δ 76,0 (dans CDCl₃)

### Fabrication du ligand 4 :

Une solution de BuLi dans l'hexane (1,6 M, 0,02 mol, 12,5 ml) et ajoutée lentement à une solution de 2,4-di terbutylphénol (4,125 g, 0,02 mol) dans le THF (20 ml) à 0°C. Le mélange est agité pendant 1 heure à température ambiante puis ajouté pendant 30 min à une solution de CgPBr (5,9022 g, 0,02 mol) dans le THF (60 ml) à 0°C. La suspension obtenue est agitée toute une nuit avant évaporation du solvant. Le ligand brut est dissous dans CH₂Cl₂ (60 ml) et 50 ml d'eau sont ajoutés. La phase aqueuse est extraite avec du CH₂Cl₂ (50 ml) et l'ensemble des phases organiques est séché sur Na₂SO₄, filtré et le solvant est évaporé. Le ligand est purifié par chromatographie sur colonne de silice en utilisant comme solvant un mélange CH₂Cl₂/Pentane (1/3 en volume) sous pression d'azote.
Quantité obtenue : 6,24 g (74%)
RMN ³¹P δ 68,8 (dans CDCl₃)

### Fabrication du ligand 5

Une solution de BuLi dans l'hexane (1,6 M, 0.02 mol, 12,5 ml) et ajoutée lentement à une solution de diphenol (1,8621 g, 0,01 mol) dans le THF (20 ml) à 0°C. Le mélange est agité pendant 1 heure à température ambiante puis ajouté pendant 30 min à une solution de CgPBr (5,9022 g, 0,02 mol) dans le THF (60 ml) à 0°C. La suspension obtenue est agitée toute une nuit avant évaporation du solvant. Le ligand brut est dissous dans CH₂Cl₂ (60 ml) et 50 ml d'eau sont ajoutés. La phase aqueuse est extraite avec du CH₂Cl₂ (50 ml) et l'ensemble des phases organiques est séché sur Na₂SO₄ et filtrée et le solvant est évaporé. Le ligand 5 est purifié par recristallisation dans un mélange CH₂Cl₂ /pentane (1/5 vol).
Quantité obtenue : 3,0 g (49%)
RMN ³¹P δ 82,8 (dans CDCl₃)

### Fabrication du ligand 6 :

Une solution de BuLi dans l'hexane (1,6 M, 0,02 mol, 12,5 ml) et ajoutée lentement à une solution de méthanol (0,5768 g, 0,018 mol) dans le THF (20 ml) à 0°C. Le mélange est agité pendant 1 heure à température ambiante puis ajouté pendant 30 min à une solution de CgPBr (5,9022 g, 0,02 mol) dans le THF (60 ml) à 0°C. La suspension obtenue est agitée toute une nuit avant évaporation du solvant. Le ligand brut est dissous dans CH₂Cl₂ (60 ml) et on 50 ml d'eau sont ajoutés. La phase aqueuse est extraite avec du CH₂Cl₂ (50 ml) et l'ensemble des phases organiques est séché sur Na₂SO₄, filtré et le solvant est évaporé. Le ligand est purifié par chromatographie sur colonne de silice en utilisant comme solvant un mélange CH₂Cl₂/Pentane (1/3 en volume) sous pression d'azote.
Quantité obtenue : 3,11 g (70%)
RMN ³¹P δ 88,5 (dans CDCl₃)

### Fabrication du ligand 7 :

Une solution de BuLi dans l'hexane (1,6 M, 0,02 mol, 12,5 ml) et ajoutée lentement à une solution de 1,2-benzenedimethanol (1,2711 g, 0,00092 mol) dans le THF (20 ml) à 0°C. Le mélange est agité pendant 1 heure à température ambiante puis ajouté pendant 30 min à une solution de CgPBr (5,9022 g, 0,02 mol) dans le THF (60 ml) à 0°C. La suspension obtenue est agitée toute une nuit avant évaporation du solvant. Le ligand brut est dissous dans CH₂Cl₂ (60 ml) et 50 ml d'eau sont ajoutés. La phase aqueuse est extraite avec du CH₂Cl₂ (50 ml) et l'ensemble des phases organiques est séché sur Na₂SO₄, filtré et le solvant est évaporé. Le ligand est purifié par chromatographie sur colonne de silice en utilisant comme solvant un mélange acétate d'éthyle/hexane (1/6 en volume) sous pression d'azote.
Quantité obtenue : 3,87 g (74%)
RMN ³P δ 85,9 et 86,0 (dans CDCl₃)

### Fabrication du ligand 8 :

Une solution de BuLi dans l'hexane (1,6 M, 0,02 mol, 12,5 ml) et ajoutée lentement à une solution de trifluoroéthanol (2,0008 g, 0,02 mol) dans le THF (20 ml) à 0°C. Le mélange est agité pendant 1 heure à température ambiante puis ajouté pendant 30 min à une solution de CgPBr (5,9022 g, 0,02 mol) dans le THF (60 ml) à 0°C. La suspension obtenue est agitée toute une nuit avant évaporation du solvant. Le ligand est purifié par chromatographie sur colonne de silice en utilisant comme solvant un mélange CH₂Cl₂/Pentane (1/3 en volume) sous pression d'azote.
Quantité obtenue : 4,81 g, (77%).
RMN ³¹P δ 97,2, ⁴J_{P,F} 7,4 Hz (dans CDCl₃)
RMN ¹⁹F δ -75,1, ⁴J_{F,P}≈ ³J_{F,H} 7,3 Hz (dans CDCl₃)

### Fabrication du ligand 9 :

Une solution de BuLi dans l'hexane (1,6 M, 0,01 mol, 6,25 ml) est ajoutée lentement à une solution de 1,2-benzène(diméthanol) (2,0725 g, 0,015 mol) dans le THF (50 ml) à 0° C. Le mélange est porté lentement à température ambiante et agité pendant 1 heure. Une solution de CgPBr (2,9511 g, 0,01 mol) dans le THF (50 ml) est ensuite ajoutée lentement à la suspension ci-dessus à 0° C pendant 30 minutes et le mélange est agité pendant 3 heures à température ambiante. Le solvant est évaporé, le solide obtenu est dissous dans CH₂Cl₂ (50 ml) et 50 ml d'eau sont ajoutés. La phase aqueuse est extraite avec 50 ml de CH₂Cl₂ et l'ensemble des phases organiques est séché sur Na₂SO₄, filtré et le solvant est évaporé. Le composé est purifié par chromatographie sur colonne de silice en utilisant comme solvant un mélange acétate d'éthyle/hexane (1/6 en volume) sous pression d'azote.
Quantité obtenue : 2,89 g (rendement 82%)
RMN ³¹P δP 87,9 (CDCl₃)

### Fabrication du ligand 10 :

Une solution de butyle lithium (BuLi) dans l'hexane (1,6 M, 0,01 mol, 6,25 ml) et ajoutée lentement à une solution de thiophénol (1,11 g, 0,01 mol) dans le tétrahydrofuranne (THF) (15 ml) à 0°C. Le mélange est agité pendant 1 heure à température ambiante puis ajouté pendant 30 min à une solution de CgPBr (2,95 g, 0,01 mol) dans le THF (50 ml) à 0°C. La suspension obtenue est agitée toute une nuit avant évaporation du solvant. Le ligand est purifié par chromatographie sur colonne de silice en utilisant comme solvant un mélange CH₂Cl₂/Pentane (1/3 en volume) sous pression d'azote.
Quantité obtenue : 2,76 g (85%)
RMN 31 P δP 21,5 (CDCI3)

### Exemples 11 à 15 : Hydroformylation du 1-hexene

Le mode opératoire général utilisé est le suivant :
Sous atmosphère inerte on charge dans un autoclave Rh(acac)(CO)₂ (6,2 mg, 0.024 mmol) le ligand conforme à l'invention dont la nature et la quantité sont indiquées dans le tableau I est 5 ml de toluène. Le réacteur est ensuite mis sous pression avec un mélange H₂/CO (1 :1 , rapport molaire) à 20 bar et porté à 60°C pendant 1 heure, puis refroidi à température ambiante, dépressurisé et purgé à l'azote. 1,2 ml de 1-hexene sont ensuite ajoutés et l'autoclave est à nouveau mis sous pression avec un mélange H₂/CO (1 :1 , rapport molaire) à 20 bar et porté à 60°C pendant 1 heure. Le réacteur est ensuite refroidi à température ambiante, dépressurisé et purgé à l'azote. Le produit obtenu est analysé par RMN ¹H (analyse par méthode de Raisonnance magnétique Nucléaire). Le produit obtenu est un mélange comprenant les produits issus de l'hydroformylation tels que : l'heptanal (produit linéaire) et le 2-méthylhexanal (produit branché)

Les résultats sont regroupés dans le tableau I suivant :

**Tableau I : exemples 11 à 15**

| exemple | ligand | Ligand/Rh (molaire) | TT(1-hexene) | Produit linéaire /branché (%molaire) |
|---|---|---|---|---|
| 11 | 1 | 4,5 | 100 | 60/40 |
| 12 | 2 | 4,5 | 100 | 65/35 |
| 13 | 3 | 4,5 | 100 | 62/38 |
| 14 | 4 | 4,5 | 100 | 66/34 |
| 15 | 6 | 4,5 | 63 | 59/41 |

### Exemples 16 à 30 : Hydrocyanation du 3-PN en AdN

Le mode opératoire général utilisé est le suivant :
Sous atmosphère d'argon, dans un tube de verre type Shott de 60 ml équipé d'un bouchon-septum, sont chargés successivement:
   - le ligand (1 mmol, 2 équivalents en P)
   - 1,21 g (15 mmol, 30 équivalents) de 3PN anhydre
   - 138 mg (0,5 mmol, 1 équivalent) de Ni(cod)₂
   - acide de Lewis (voir tableau 2 pour quantité)

Le mélange est porté sous agitation à 70°C. La cyanhydrine de l'acétone est injectée dans le milieu réactionnel par un pousse-seringue à un débit de 0,45 ml par heure. Après 3 heures d'injection le poussé seringue est arrêté. Le mélange est refroidi à température ambiante, dilué à l'acétone et analysé par chromatographie en phase gazeuse.

Les résultats sont regroupés dans le tableau suivant :

**Tableau II: exemples 16 à 30**

| exemple | ligand | Acide de Lewis | Acide de Lewis/Ni (molaire) | Linéarité | RR (DN) |
|---|---|---|---|---|---|
| 16 | 1 | Ph₂BOBPh₂ | 0,5 | 73,1 | 3,2 |
| 17 | 5 | ZnCl₂ | 1 | 99 | 1,3 |
| 18 | 5 | BPh₃ | 1 | 69,8 | 4,4 |
| 19 | 5 | Ph₂BOBPh₂ | 0,5 | 79 | 4,5 |
| 20 | 6 | BPh₃ | 1 | 67 | 4,6 |
| 21 | 6 | Ph₂BOBPh₂ | 0,5 | 78,7 | 7,4 |
| 22 | 6 | TIBAO | 0,5 | 64,2 | 18,5 |
| 23 | 7 | ZnCl₂ | 1 | 74,7 | 39,9 |
| 24 | 7 | Ph₂BOBPh₂ | 0,5 | 73,2 | 5,9 |
| 25 | 8 | Ph2BOBPh2 | 0,5 | 75,1 | 15,2 |
| 26 | 8 | TIBAO | 0,5 | 60,8 | 36,3 |
| 27 | 9 | ZnCl₂ | 1 | 93,7 | 0,8 |
| 28 | 9 | TIBAO | 2 | 71 | 6,8 |
| 29 | 10 | ZnCl₂ | 1 | 100 | 0,6 |
| 30 | 10 | TIBAO | 0,5 | 74 | 0,8 |

## Revendications

1. Composés organophosphorés **caractérisé en ce qu'**ils correspondent à l'une des formules générales (I) ou (II): 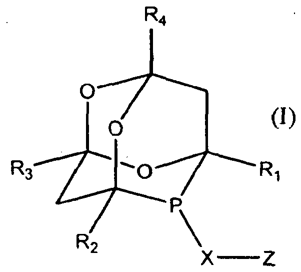 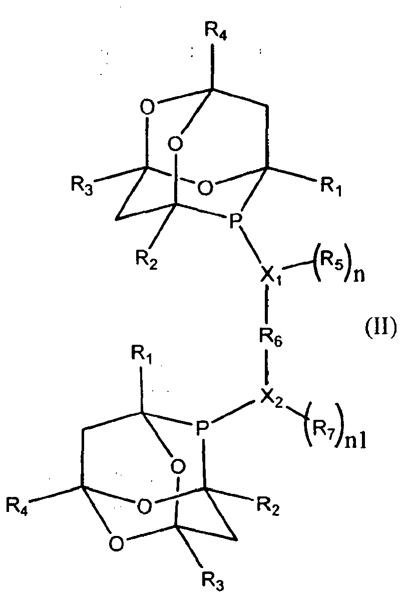 dans lesquelles :
- R₁, R₂, R₃, R₄, R₅, R₇ et Z identiques ou différents représentent un atome d'hydrogène, un radical alkyl linéaire ou ramifié ayant de 1 à 12 carbones pouvant contenir des hétéroatomes, un radicale comprenant un radical aromatique ou cycloaliphatique substitué ou non pouvant comprendre des hétéroatomes, un radical carbonyle, alcoxycarbonyle ou alcoxy, un atome d'halogène, un groupement nitrile ou un groupement halogénoalkyle ayant 1 à 12 atomes de carbone
- X, X1 et X2 identiques ou différents représentent un atome d'oxygène, d'azote, de soufre, ou de silicium
- R₆ représente une liaison covalente, un radical aliphatique linéaire ou ramifié, un radical comprenant un cycle aromatique ou cycloaliphatique substitué ou non ou plusieurs cycles aromatiques condensés ou reliés entre eux par une liaison
- n et n1 identiques ou différents sont des nombres entiers respectivement égaux à la valence des éléments X₁, X₂ diminuée de 2.

2. Composés selon la revendication 1, **caractérisés en ce qu'**ils appartiennent à la famille des organophosphinites et qu'ils correspondent à l'une des formules générales (III) ou (IV): 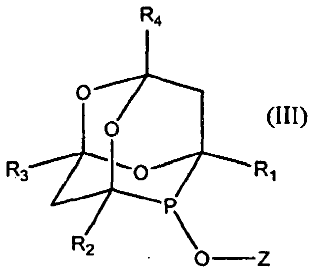 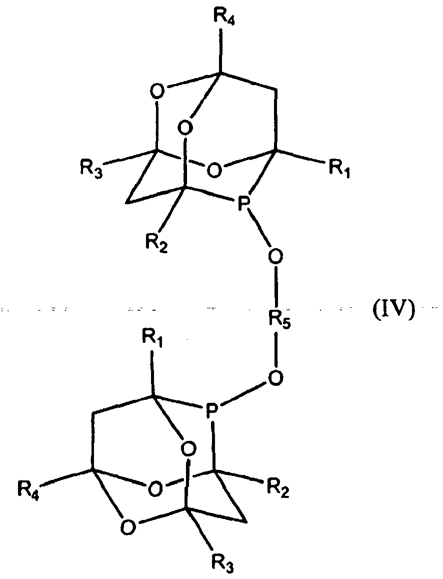 dans lesquelles :
- R₁, R₂, R₃, R₄, identiques ou différents représentent un atome d'hydrogène, un radical alkyl linéaire ou ramifié ayant de 1 à douze atomes de carbone pouvant contenir des hétéroatomes,
- Z représente un radical aromatique ou cycloaliphatique substitué ou non pouvant comprendre des hétéroatomes, un radical carbonyle, alcoxycarbonyle ou alcoxy, un atome d'halogène, un groupement nitrile ou un groupement halogénoalkyle ayant un à douze atomes de carbone,
- R₅ représente une liaison covalente, un radical aliphatique linéaire ou ramifié, un radical comprenant un cycle aromatique ou cycloaliphatique substitué ou non ou plusieurs cycles aromatiques condensés ou reliés entre eux par une liaison.

3. Composés selon la revendication 1 ou 2, **caractérisés en ce qu'**ils sont choisis dans le groupe comprenant les composés de formules suivantes :

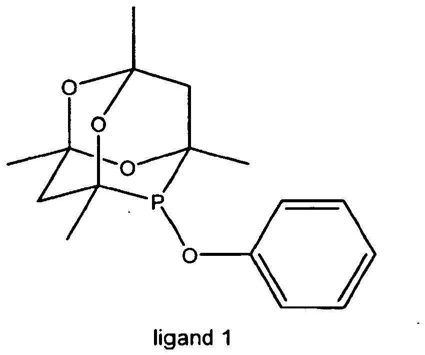

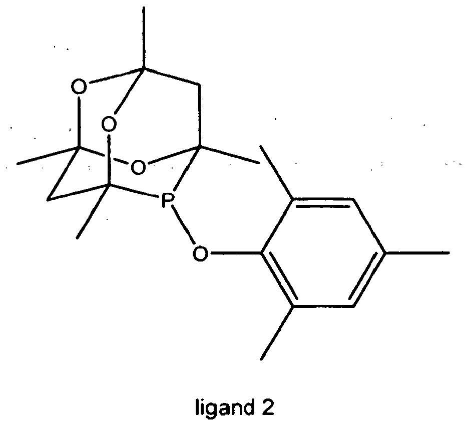

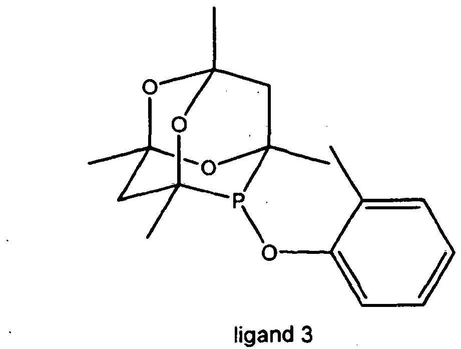

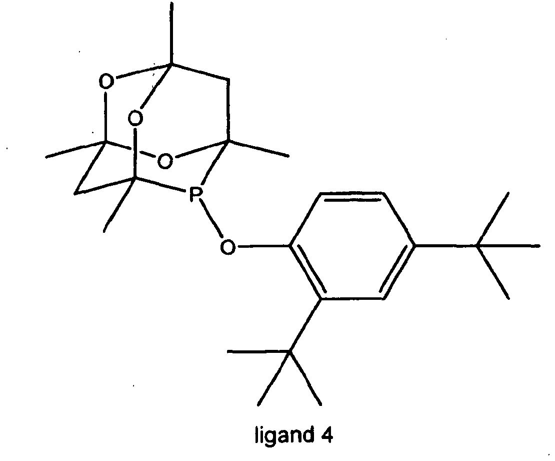

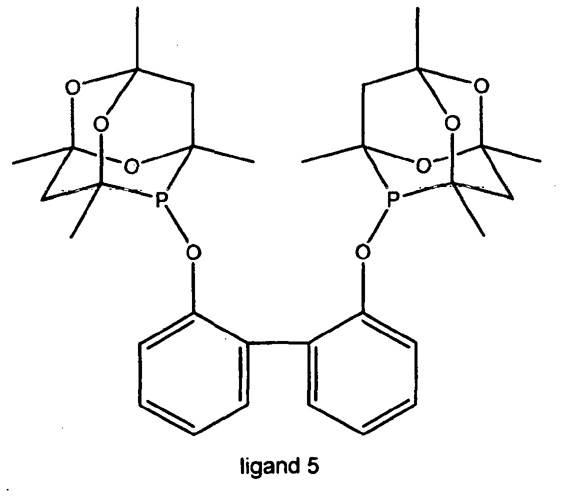

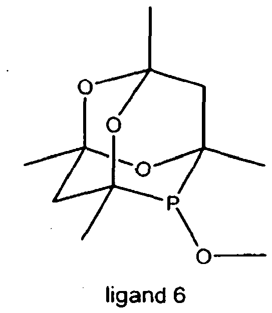

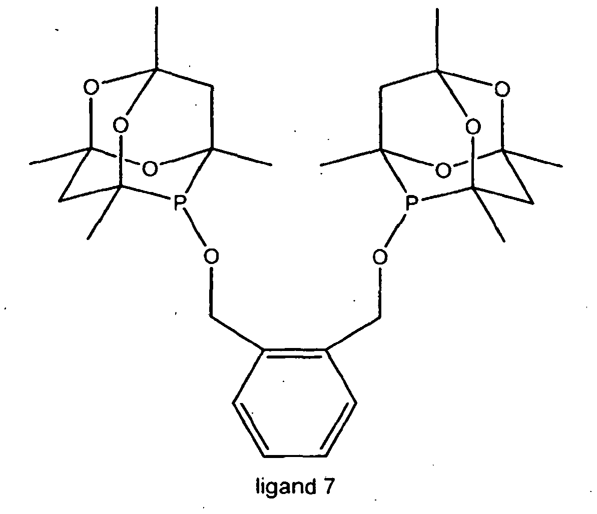

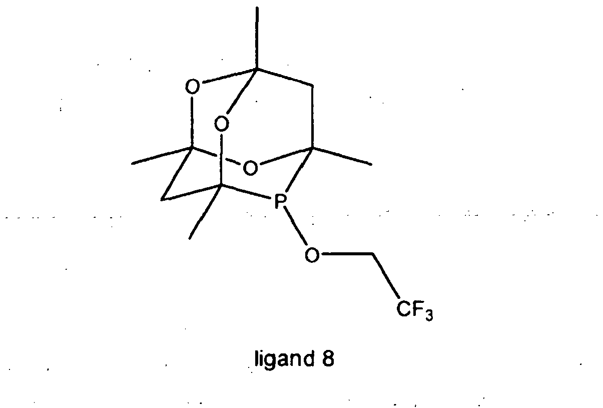

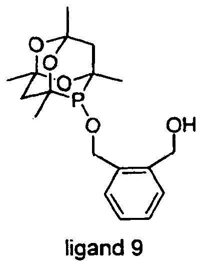

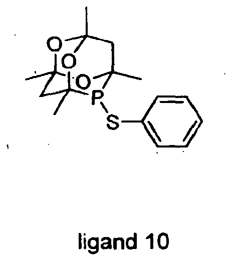

4. Système catalytique comprenant un élément métallique formant un complexe avec un composé organophosphoré selon l'une des revendications 1 à 3, **caractérisé en ce que** le complexe correspond aux formules générale (V) ou (VI) suivantes :
M [L_{f}]ₜ (V)
HM [L_{f}]ₜ₊ₙCO₄₋ₙ (VI)
Dans laquelle:
M est un métal de transition
L_{f} représente au moins un ligand organophosphoré de formule (I), (II), (III) ou (IV) t représente un nombre compris entre 1 et 10 (bornes incluses)
n représente un nombre compris entre 1 et 4 (bornes incluses)

5. Système selon la revendication 4, **caractérisé en ce que** l'élément métallique M est choisi dans le groupe comprenant le nickel, le cobalt, le fer, le ruthénium, le rhodium, le palladium, l'osmium, l'iridium, le platine, le cuivre, l'argent, l'or, le zinc, le cadmium, le mercure.

6. Procédé d'hydrocyanation d'un composé hydrocarboné comprenant au moins une insaturation éthylénique par réaction en milieu liquide avec le cyanure d'hydrogène en présence d'un système catalytique selon la revendication 4 ou 5 **caractérisé en ce que** l'élément métallique est le nickel.

7. Procédé selon la revendication 6, **caractérisé en ce que** les composés organiques comportant au moins une insaturation éthylénique sont choisis parmi les dioléfines comme le butadiène, l'isoprène, l'hexadiène-1,5, le cyclooctadiène-1,5, les nitriles aliphatiques à insaturation éthylénique, particulièrement les pentènenitriles linéaires comme le pentène-3-nitrile, le pentène-4-nitrile, les monooléfines comme le styrène, le méthyl-styrène, le vinyl-naphtalène, le cyclohexène, le méthyl-cyclohexène ainsi que les mélanges de plusieurs de ces composés.

8. Procédé selon la revendication 6 ou 7, **caractérisé en ce que** la quantité de composé du nickel est choisie de telle sorte qu'il y ait par mole de composé organique à hydrocyaner ou isomériser entre 10⁻⁴ et 1 mole de nickel ou de l'autre métal de transition mis en oeuvre et **en ce que** la quantité de composés organosphosphorés utilisée est choisie de telle sorte que le nombre de moles de ces composés rapporté à 1 mole de métal de transition soit de 0,5 à 100.

9. Procédé selon l'une des revendications 6 à 8 , **caractérisé en ce que** les composés à insaturation éthyléniques sont des composés nitriles à insturation éthyléniques et que l'on opère en présence d'un système catalytique comprenant au moins un composé d'un métal de transition, au moins un composé de formule (I), (II), (III) ou (IV) et un cocatalyseur consistant en au moins un acide de Lewis.

10. Procédé selon la revendication 9, **caractérisé en ce que** les composés nitriles à insaturation éthylénique sont choisis parmi les nitriles aliphatiques à insaturation éthylénique comprenant les pentènenitriles linéaires comme le pentène-3-nitrile, le pentène-4-nitrile et leurs mélanges.

11. Procédé selon l'une des revendications 9 à 10, **caractérisé en ce que** l'acide de Lewis mis en oeuvre comme cocatalyseur est choisi parmi les composés des éléments des groupes Ib, IIb, IIIa, IIIb, IVa, IVb, Va, Vb, Vlb, VIIb et VIII de la Classification périodique des éléments.

12. Procédé selon l'une des revendications 9 à 11, **caractérisé en ce que** l'acide de Lewis est choisi parmi le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le trifluorométhylsulfonate d'indium, les chlorures ou bromures des éléments des terres rares comme le lanthane, le cérium, le praséodyme, le néodyme, le samarium, l'europium, le gadolinium, le terbium, le dysprosium, l'hafnium, l'erbium, le thallium, l'ytterbium et le lutétium, le chlorure de cobalt, le chlorure ferreux, le chlorure d'yttrium et leurs mélanges, les composés organométalliques.

13. Procédé selon l'une des revendications 6 à 12, **caractérisé en ce que** l'on réalise en absence de cyanure d'hydrogène l'isomérisation en pentènenitriles, du méthyl-2-butène-3-nitrile présent dans le mélange réactionnel provenant de l'hydrocyanation du butadiène, en opérant en présence d'un catalyseur comportant au moins un composé de formule (I), (II), (III) ou (IV) et au moins un composé d'un métal de transition.

14. Procédé d'hydroformylation d'alcènes en présence d'un système catalytique selon la revendication 4 ou 5 **caractérisé en ce que** l'élément métallique est le rhodium ou le cobalt.

## Patentansprüche

1. Organophosphorverbindungen, **dadurch gekennzeichnet, dass** sie einer der allgemeinen Formeln (I) oder (II) entsprechen: 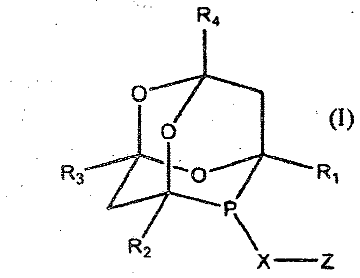 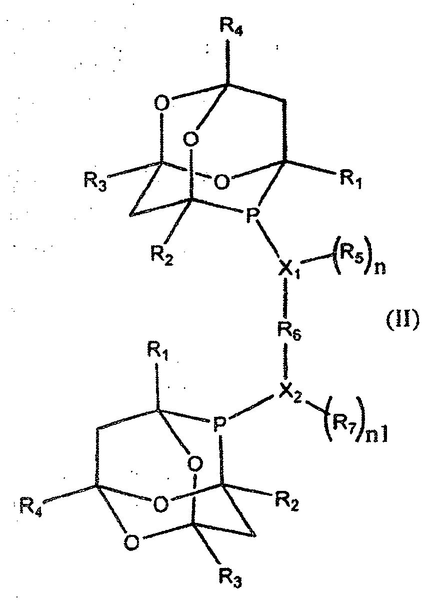 worin:
- R₁, R₂, R₃, R₄, R₅, R₇ und Z gleich oder Verschieden sind und für ein Wasserstoffatom, einen linearen oder verzweigten Alkylrest mit 1 bis 12 Kohlenstoffatomen, der Heteroatome enthalten kann, einen Rest, der einen substituierten oder unsubstituierten aromatischen oder cycloaliphatischen Rest, der Heteroatome enthalten kann, umfasst, einen Carbonyl-, Alkoxycarbonyl- oder Alkoxyrest, ein Halogenatom, eine Nitrilgruppe oder eine Halogenalkylgruppe mit 1 bis 12 Kohlenstoffatomen stehen, X, X1 und X2 gleich oder verschieden sind und für ein Sauerstoff-, Stickstoff-, Schwefel- oder Siliciumatom stehen,
- R₆ für eine kovalente Bindung, einen linearen oder verzweigten aliphatischen Rest, einen Rest, der einen substituierten oder unsubstituierten aromatischen oder cycloaliphatischen Ring oder mehrere kondensierte oder miteinander über eine Bindung verbundene aromatische Ringe umfasst, steht,
- n und n1 gleich oder verschieden sind und für ganze Zahlen stehen, die der um 2 verringerten Wertigkeit der Elemente X₁ bzw. X₂ entsprechen.

2. Verbindungen nach Anspruch 1, **dadurch gekennzeichnet, dass** sie zur Organophosphinit-Familie gehören und einer der allgemeinen Formeln (III) oder (IV) entsprechen: 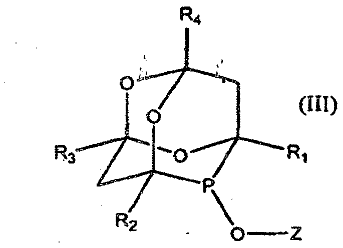 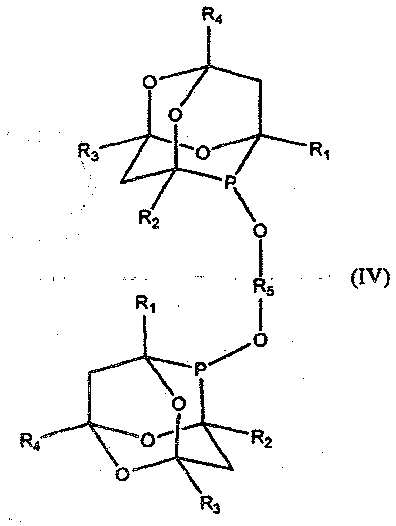 worin:
- R₁, R₂, R₃, R₄ gleich oder verschieden sind und für ein Wasserstoffatom oder einen linearen oder verzweigten Alkylrest mit 1 bis zwölf Kohlenstoffatomen, der Heteroatome enthalten kann, stehen,
- Z für einen substituierten oder unsubstituierten aromatischen oder cycloaliphatischen Rest, der Heteroatome enthalten kann, einen Carbonyl-, Alkoxycarbonyl- oder Alkoxyrest, ein Halogenatom, eine Nitrilgruppe oder eine Halogenalkylgruppe mit einem bis zwölf Kohlenstoffatomen steht,
- R₅ für eine kovalente Bindung, einen linearen oder verzweigten aliphatischen Rest, einen Rest, der einen substituierten oder unsubstituierten aromatischen oder cycloaliphatischen Ring oder mehrere kondensierte oder miteinander über eine Bindung verbundene aromatische Ringe umfasst, steht.

3. Verbindungen nach Anspruch 1 oder 2, **dadurch gekennzeichnet, dass** sie aus der Gruppe ausgewählt sind, die die Verbindungen der folgenden Formeln umfasst:

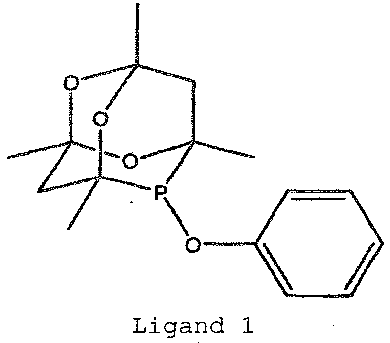

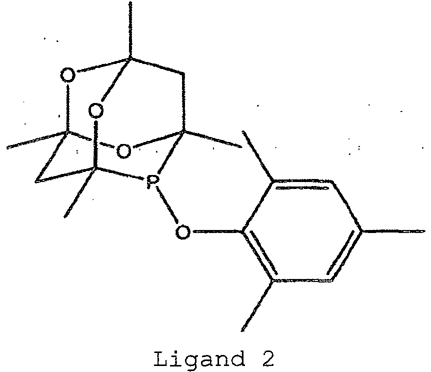

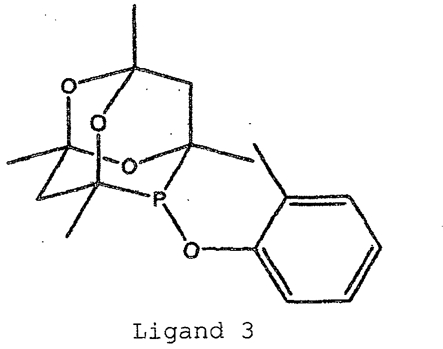

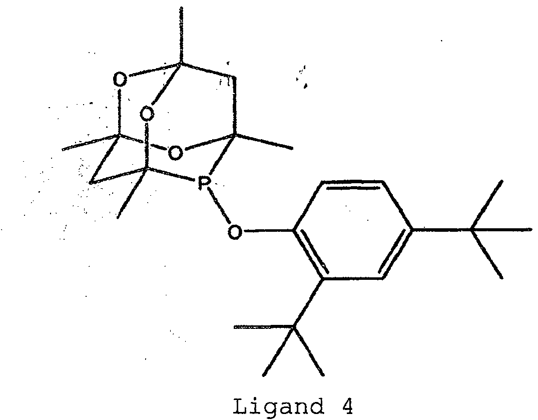

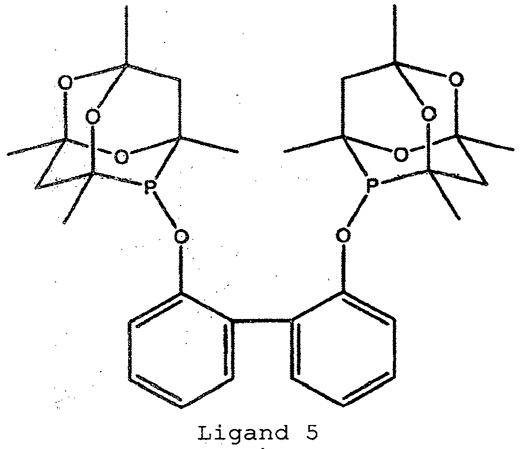

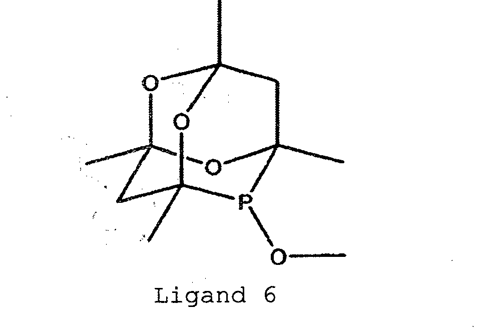

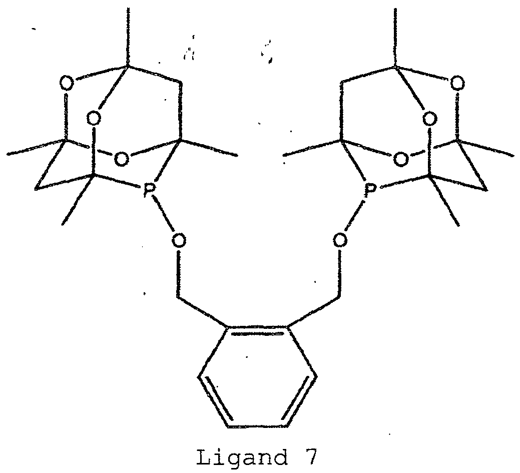

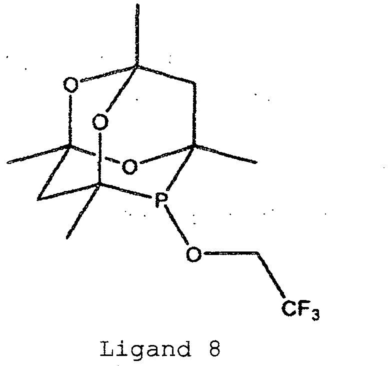

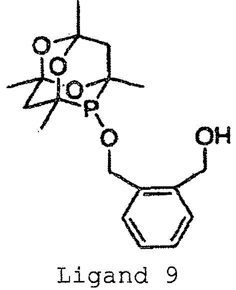

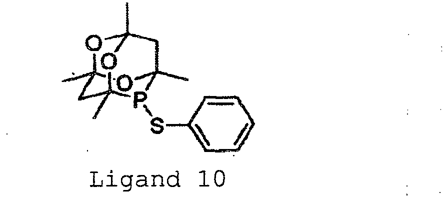

4. Katalytisches System, umfassend ein Metallelement, das mit einer Organophosphorverbindung nach einem der Ansprüche 1 bis 3 einen Komplex bildet, **dadurch gekennzeichnet, dass** der Komplex den folgenden allgemeinen Formeln (V) oder (VI) entspricht:
M [L_{f}]ₜ (V)
HM [L_{f}]ₜ₊ₙCO₄₋ₙ (VI)
worin:
M für ein Übergangsmetall steht,
L_{f} für mindestens einen Organophosphorliganden der Formel (I), (II), (III) oder (IV) steht,
t für eine Zahl zwischen 1 und 10 (Grenzen eingeschlossen) steht,
n für eine Zahl zwischen 1 und 4 (Grenzen eingeschlossen) steht.

5. System nach Anspruch 4, **dadurch gekennzeichnet, dass** das Metallelement M aus der Gruppe umfassend Nickel, Cobalt, Eisen, Ruthenium, Rhodium, Palladium, Osmium, Iridium, Platin, Kupfer, Silber, Gold, Zink, Cadmium, Quecksilber ausgewählt ist.

6. Verfahren zur Hydrocyanierung einer Kohlenwasserstoffverbindung mit mindestens einer ethylenischen Ungesättigtheit durch Umsetzung mit Cyanwasserstoff in flüssigem Medium in Gegenwart eines katalytischen Systems nach Anspruch 4 oder 5, dadurch gekenntzeichnet, dass es sich bei dem Metallelement um Nickel handelt.

7. Verfahren nach Anspruch 6, **dadurch gekennzeichnet, dass** man die organischen Verbindungen mit mindestens einer ethylenischen Ungesättigtheit aus Diolefinen wie Butadien, Isopren, 1,5-Hexadien, 1,5-Cyclooctadien, ethylenisch ungesättigten aliphatischen Nitrilen, insbesondere linearen Pentennitrilen wie 3-Pentennitril und 4-Pentennitril, Monoolefinen wie Styrol, Methylstyrol, Vinylnaphthalin, Cyclohexen, Methylcyclohexen und Mischungen mehrerer dieser Verbindungen auswählt.

8. Verfahren nach Anspruch 6 oder 7, **dadurch gekennzeichnet, dass** man die Menge an Nickelverbindung so wählt, dass pro Mol zu hydrocyanierende oder isomerisierende organische Verbindung zwischen 10⁻⁴ und 1 mol verwendetes Nickel oder anderes Übergangsmetall vorliegen, und die verwendete Menge an Organophosphorverbindungen so wählt, dass die Zahl der Mole dieser Verbindungen, bezogen auf 1 mol Übergangsmetall, 0,5 bis 100 beträgt.

9. Verfahren nach einem der Ansprüche 6 bis 8, **dadurch gekennzeichnet, dass** es sich bei den Verbindungen mit ethylenischer Ungesättigtheit um Nitrilverbindungen mit ethylenischer Ungesättigtheit handelt und man in Gegenwart eines katalytischen Systems, das mindestens eine Übergangsmetallverbindung, mindestens eine Verbindung der Formel (I), (II), (III) oder (IV) und einen Cokatalysator, der mindestens aus einer Lewis-Säure besteht, umfasst, arbeitet.

10. Verfahren nach Anspruch 9, **dadurch gekennzeichnet, dass** man die Nitrilverbindungen mit ethylenischer Ungesättigtheit aus aliphatischen Nitrilen mit ethylenischer Ungesättigtheit, die lineare Pentennitrile wie 3-Pentennitril, 4-Pentennitril und Mischungen davon umfassen, auswählt.

11. Verfahren nach einem der Ansprüche 9 bis 10, **dadurch gekennzeichnet, dass** man die als Cokatalysator verwendete Lewis-Säure aus den Verbindungen der Gruppen Ib, IIb, IIIa, IIIb, IVa, IVb, Va, Vb, VIb, VIIb und VIII des Periodensystems der Elemente auswählt.

12. Verfahren nach einem der Ansprüche 9 bis 11, **dadurch gekennzeichnet, dass** man die Lewis-Säure aus Zinkchlorid, Zinkbromid, Zinkiodid, Manganchlorid, Manganbromid, Cadmiumchlorid, Cadmiumbromid, Zinn (II) -chlorid, Zinn (II) -bromid, Zinn(II)-sulfat, Zinn(II)-tartrat, Indiumtrifluormethylsulfonat, den Chloriden oder Bromiden der Seltenerdmetalle wie Lanthan, Cer, Praseodym, Neodym, Samarium, Europium, Gadolinium, Terbium, Dysprosium, Hafnium, Erbium, Thallium, Ytterbium und Lutetium, Cobaltchlorid, Eisen(II)-chlorid, Yttriumchlorid und Mischungen davon und Organometallverbindungen auswählt.

13. Verfahren nach einem der Ansprüche 6 bis 12, **dadurch gekennzeichnet, dass** man das in der aus der Hydrocyanierung von Butadien stammenden Reaktionsmischung vorliegende 2-Methyl-3-butennitril in Abwesenheit von Cyanwasserstoff und in Gegenwart eines Katalysators, der mindestens eine Verbindung der Formel (I), (II), (III) oder (IV) und mindestens eine Übergangsmetallverbindung enthält, zu Pentennitrilen isomerisiert.

14. Verfahren zur Hydroformylierung von Alkenen in Gegenwart eines katalytischen Systems nach Anspruch 4 oder 5, **dadurch gekennzeichnet, dass** es sich bei dem Metallelement um Rhodium oder Cobalt handelt.

## Claims

1. Organophosphorus compounds, **characterized in that** they correspond to one of the general formulae (I) or (II): 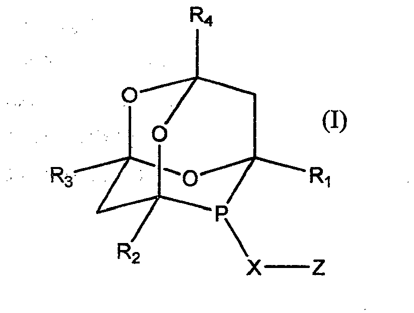 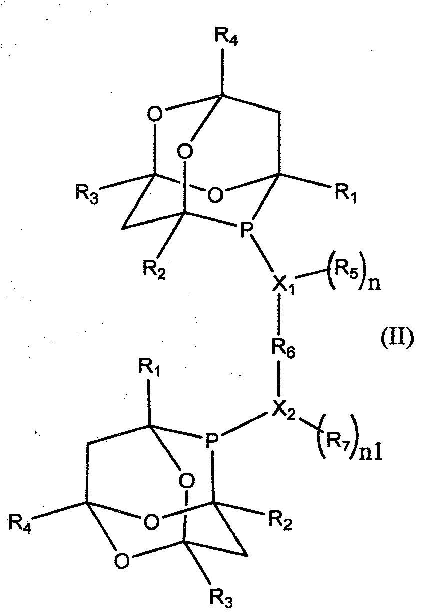 in which:
- R₁, R₂, R₃, R₄, R₅, R₇ and Z, which may be identical or different, represent a hydrogen atom, a linear or branched alkyl radical having from 1 to 12 carbons which can contain heteroatoms, a radical comprising a substituted or unsubstituted aromatic or cycloaliphatic radical which can comprise heteroatoms, a carbonyl, alkoxycarbonyl or alkoxy radical, a halogen atom, a nitrile group or a haloalkyl group having 1 to 12 carbon atoms
- X, X1 and X2, which may be identical or different, represent an oxygen, nitrogen, sulphur or silicon atom
- R₆ represents a covalent bond, a linear or branched aliphatic radical, a radical comprising a substituted or unsubstituted aromatic or cycloaliphatic ring or several aromatic rings, condensed or joined together by a bond
- n and n1, which may be identical or different, are integers respectively equal to the valency of elements X₁, X₂ reduced by 2.

2. Compounds according to Claim 1, **characterized in that** they belong to the family of the organophosphinites and that they correspond to one of the general formulae (III) or (IV): 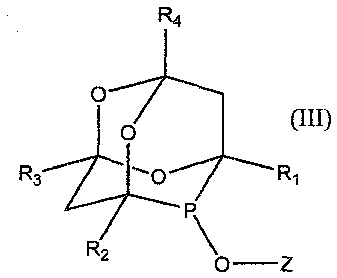 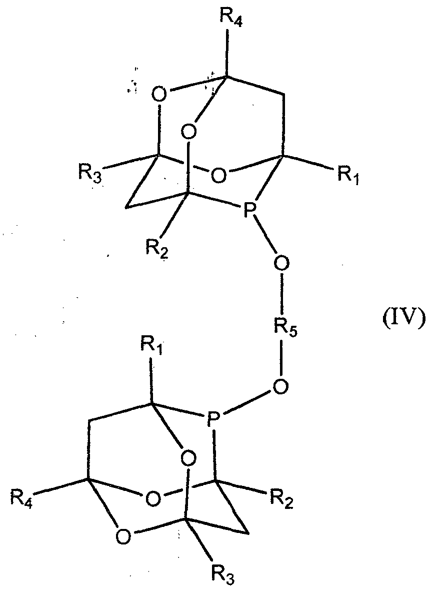 in which:
- R₁, R₂, R₃, R₄, which may be identical or different, represent a hydrogen atom, a linear or branched alkyl radical having from 1 to 12 carbon atoms which can contain heteroatoms,
- Z represents a substituted or unsubstituted aromatic or cycloaliphatic radical which can comprise heteroatoms, a carbonyl, alkoxycarbonyl or alkoxy radical, a halogen atom, a nitrile group or a haloalkyl group having 1 to 12 carbon atoms,
- R₅ represents a covalent bond, a linear or branched aliphatic radical, a radical comprising a substituted or unsubstituted aromatic or cycloaliphatic ring or several aromatic rings, condensed or joined together by a bond.

3. Compounds according to Claim 1 or 2, **characterized in that** they are selected from the group comprising the compounds with the following formulae:

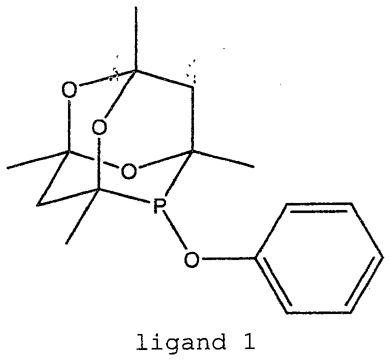

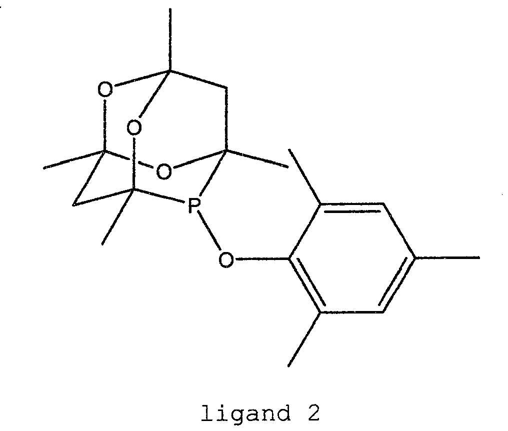

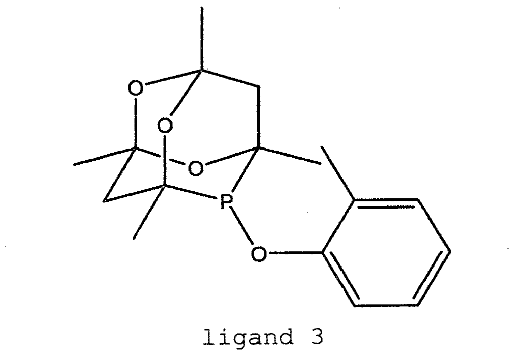

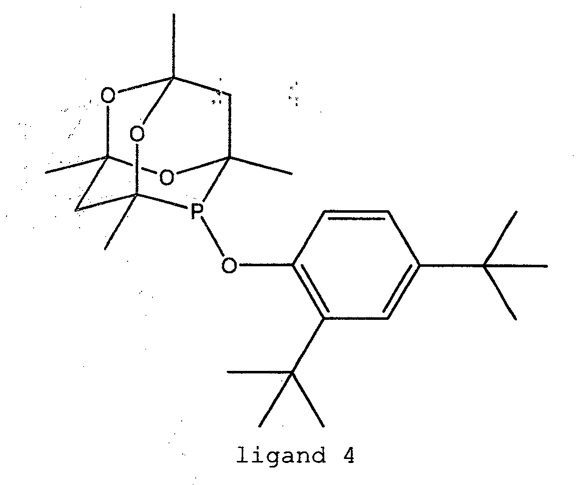

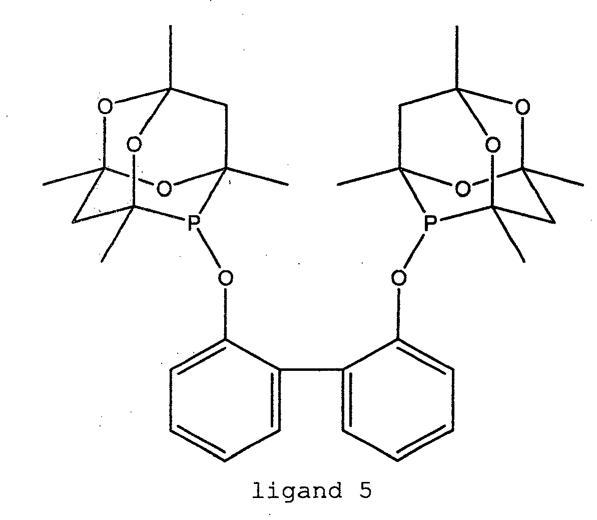

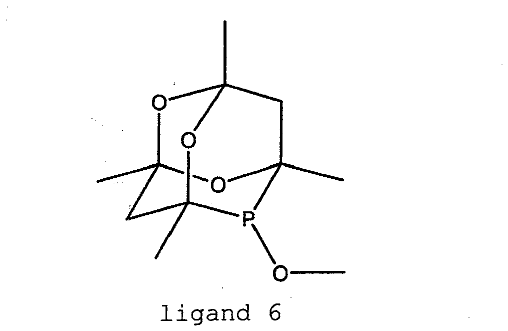

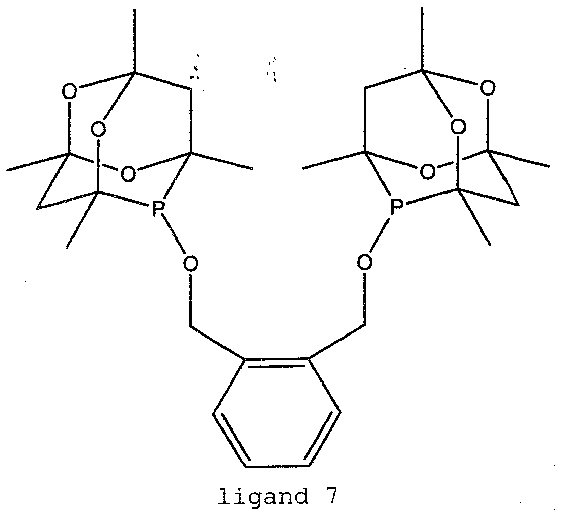

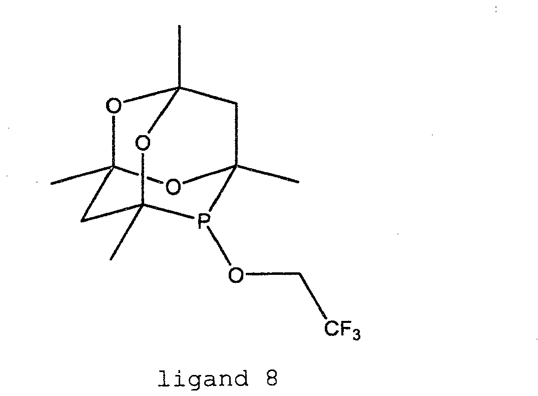

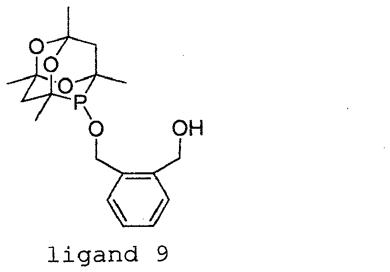

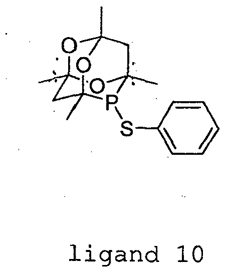

4. Catalytic system comprising a metallic element forming a complex with an organophosphorus compound according to one of Claims 1 to 3, **characterized in that** the complex corresponds to the following general formulae (V) or (VI):
M [L_{f}]ₜ t (V)
HM [L_{f}]ₜ₊ₙCO₄₋ₙ (VI)
in which:
M is a transition metal
L_{f} represents at least one organophosphorus ligand of formula (I), (II), (III) or (IV) t represents a number between 1 and 10 (inclusive) n represents a number between 1 and 4 (inclusive)

5. System according to Claim 4, **characterized in that** the metallic element M is selected from the group comprising nickel, cobalt, iron, ruthenium, rhodium, palladium osmium, iridium, platinum, copper, silver, gold, zinc, cadmium, mercury.

6. Method of hydrocyanation of a hydrocarbon compound comprising at least one ethylenic unsaturation by reaction in a liquid medium with hydrogen cyanide in the presence of a catalytic system according to Claim 4 or 5, **characterized in that** the metallic element is nickel.

7. Method according to Claim 6, **characterized in that** the organic compounds bearing at least one ethylenic unsaturation are selected from the diolefins such as butadiene, isoprene, hexadiene-1,5, cyclooctadiene-1,5, ethylenically unsaturated aliphatic nitriles, particularly linear pentenenitriles such as pentene-3-nitrile, pentene-4-nitrile, monoolefins such as styrene, methylstyrene, vinylnaphthalene, cyclohexene, methylcyclohexene as well as mixtures of several of these compounds.

8. Method according to Claim 6 or 7, **characterized in that** the amount of nickel compound is selected in such a way that there is, per mol of organic compound that is to undergo hydrocyanation or isomerization, between 10⁻⁴ and 1 mol of nickel or of the other transition metal employed and **in that** the amount of organophosphorus compounds used is selected in such a way that the number of moles of these compounds relative to 1 mol of transition metal is from 0.5 to 100.

9. Method according to one of Claims 6 to 8, **characterized in that** the ethylenically unsaturated compounds are ethylenically unsaturated nitrile compounds and **in that** it is carried out in the presence of a catalytic system comprising at least one compound of a transition metal, at least one compound of formula (I), (II), (III) or (IV) and a cocatalyst consisting of at least one Lewis acid.

10. Method according to Claim 9, **characterized in that** the ethylenically unsaturated nitrile compounds are selected from the ethylenically unsaturated aliphatic nitriles comprising linear pentenenitriles such as pentene-3-nitrile, pentene-4-nitrile and mixtures thereof.

11. Method according to one of Claims 9 to 10, **characterized in that** the Lewis acid employed as cocatalyst is selected from compounds of the elements of groups Ib, IIb, IIIa, IIIb, IVa, IVb, Va, Vb, VIb, VIIb and VIII of the periodic system.

12. Method according to one of Claims 9 to 11, **characterized in that** the Lewis acid is selected from zinc chloride, zinc bromide, zinc iodide, manganese chloride, manganese bromide, cadmium chloride, cadmium bromide, stannous chloride, stannous bromide, stannous sulphate, stannous tartrate, indium trifluoromethylsulphonate, chlorides or bromides of rare earth elements such as lanthanum, cerium, praseodymium, neodymium, samarium, europium, gadolinium, terbium, dysprosium, hafnium, erbium, thallium, ytterbium and lutetium, cobalt chloride, ferrous chloride, yttrium chloride and mixtures thereof, organometallic compounds.

13. Method according to one of Claims 6 to 12, **characterized in that** the isomerization, to pentenenitriles, of the methyl-2-butene-3-nitrile present in the reaction mixture resulting from the hydrocyanation of butadiene, is carried out in the absence of hydrogen cyanide, working in the presence of a catalyst bearing at least one compound of formula (I), (II), (III) or (IV) and at least one compound of a transition metal.

14. Method of hydroformylation of alkenes in the presence of a catalytic system according to Claim 4 or 5, **characterized in that** the metallic element is rhodium or cobalt.
